# EUROPEAN PATENT SPECIFICATION

(11) **EP 3 268 488 B1**
(45) Date of publication and mention of the grant of the patent: **06.11.2019**
(21) Application number: 16762365.1
(22) Date of filing: 09.03.2016
(51) Int. Cl.: C12Q 1/04, C12Q 1/68, C12Q 1/6883, C12Q 1/6881

(54) **METHOD FOR IDENTIFYING DISEASE-ASSOCIATED CDR3 PATTERNS IN AN IMMUNE REPERTOIRE**
VERFAHREN ZUR IDENTIFIZIERUNG VON KRANKHEITSASSOZIIERTEN CDR3-MUSTERN IN EINEM IMMUNREPERTOIRE
PROCÉDÉ D'IDENTIFICATION DE MOTIFS CDR3 ASSOCIÉS À UNE MALADIE DANS UN RÉPERTOIRE IMMUNITAIRE

(30) Priority: 09.03.2015 US 201562130512 P
(43) Date of publication of application: 17.01.2018
(73) Proprietor: Irepertoire, Inc., Huntsville, AL 35806 (US)
(72) Inventor: HAN, Jian, Huntsville, AL 35802 (US)
(74) Representative: Dehns
(86) International application number: PCT/US2016/021430
(87) International publication number: WO 2016/144996

(56) References cited:
- WO-A1-2013/055789
- WO-A1-2013/086450
- WO-A1-2014/124451
- WO-A2-2011/106738
- CN-A- 101 225 441
- C. WANG ET AL: "High throughput sequencing reveals a complex pattern of dynamic interrelationships among human T cell subsets", PROCEEDINGS OF THE NATIONAL ACADEMY OF SCIENCES, vol. 107, no. 4, 4 January 2010 (2010-01-04), pages 1518-1523, XP055114155, ISSN: 0027-8424, DOI: 10.1073/pnas.0913939107
- DMITRY A. BOLOTIN ET AL: "Next generation sequencing for TCR repertoire profiling: Platform-specific features and correction algorithms", EUROPEAN JOURNAL OF IMMUNOLOGY, vol. 42, no. 11, 24 September 2012 (2012-09-24), pages 3073-3083, XP055235351, ISSN: 0014-2980, DOI: 10.1002/eji.201242517
- DENGRUI LI ET AL: "Profiling the T-cell receptor repertoire of patient with pleural tuberculosis by high-throughput sequencing", IMMUNOLOGY LETTERS., vol. 162, no. 1, 1 November 2014 (2014-11-01), pages 170-180, XP055486783, NL ISSN: 0165-2478, DOI: 10.1016/j.imlet.2014.08.012
- ROBINSON, W. H.: 'Sequencing the functional antibody repertoire - diagnostic and therapeutic discovery' NAT REV RHEUMATOL. vol. 11, no. 3, 23 December 2014, pages 171 - 182, XP055310136
- YANG ET AL.: 'Distinct mechanisms define murine B cell lineage immunoglobulin heavy chain (IgH) repertoires' ELIFE vol. 4, 30 September 2015, pages 1 - 31, XP055310142

## Description

### Field of the Invention

The invention relates to methods for recognizing disease-associated immune repertoires in human and/or animal subjects and for the use of such methods for disease diagnosis and for the study of disease processes.

### Background of the Invention

The diverse antigen receptors of T and B lymphocytes are produced by somatic recombination of a limited, but large number of gene segments. These gene segments - V (variable), D (diversity), J (joining), and C (constant) - determine the binding specificity and downstream applications of immunoglobulins and T cell receptors (TCRs). The rearranged V(D)J portion of the receptor, termed the V-region, is of great interest, because it is responsible for epitope recognition. When the V(D)J is translated into an amino acid sequence, the V-region can be subdivided into several parts consisting of the leader sequence, framework (FR) 1, complementarity-determining region 1 (CDR1), FR2, CDR2, FR3, CDR3, FR4, and the C-domains.

The CDR3 is of particular interest because studies have indicated that this region is associated with antigen-specificity. Compared to normal subjects, patients with various diseases may experience quantitative and/or qualitative changes in their immune repertoire. Quantitative changes may be apparent as increases and decreases in immune repertoire diversity. Qualitative changes may present as increased sharing of disease-specific CDR3s in T or B cells.

The immune system mounts a response to various conditions, such as cancer, bacterial infections, viral infections, and fungal infections. Further, in some subjects it may actually produce a deleterious response to the tissues of the body, resulting in, for example, autoimmune disease or rejection of grafts. The types and degrees of these immune responses could, if accurately accessed, potentially be one of the most important and accurate indicators of the presence or absence of a particular disease or of undesirable immune responses.

Humans, however, are estimated to have as many as 10¹⁵-10²⁵ different T cells, due to the number of possible VDJ rearrangements, n-additions, and alpha-beta chain combinations. Assuming that, for a particular disease, there are only 10³ disease-specific CDR3s that may quantitatively (through up or down regulation) or qualitatively (through gain or loss) change, the signal-to-noise ratio in these circumstances is very weak. Therefore, conventional methods are impractical to assess this information for diagnostic purposes.

What are needed are improved methods for immune response assessment and for the development of diagnostic tests based on such immune response and the resulting immune repertoire.

### Summary of the Invention

In one embodiment, the present disclosure relates to a method for developing a diagnostic test using the immune repertoire, the method comprising the steps of: (a) collecting a sample from each of multiple subjects in a patient group and a control group, wherein the patient group comprises subjects who have the same disease and the control group comprises subjects who are categorized as healthy; (b) amplifying and sequencing the immune repertoire of each subject in each of the two groups to identify each unique CDR3 sequence present in the sample and to determine the frequency of occurrence of each unique CDR3 sequence; (c) identifying CDR3 sequences that are shared between at least two subjects in each of the control group and the patient group; (d) ranking the identified CDR3 sequences by order of frequency of occurrence; (e) identifying Linklets from each group; and (f) identifying the Linklets that are associated to a statistically significant degree with the patient group to provide a disease signature.

In certain embodiments, the sample is peripheral blood. In other embodiments, the sample is tissue. In certain embodiments, fewer than 1,000 CDR3 sequences are identified as shared between at least two subjects. In other embodiments, at least 1,000 CDR3 sequences are identified as shared between at least two subjects. In certain embodiments, at least about 10⁶ Linklets are identified from each group. In other embodiments, fewer than 10⁶ Linklets are identified from each group.

### Brief Description of the Figures

The disclosure can be better understood with reference to the following figures.

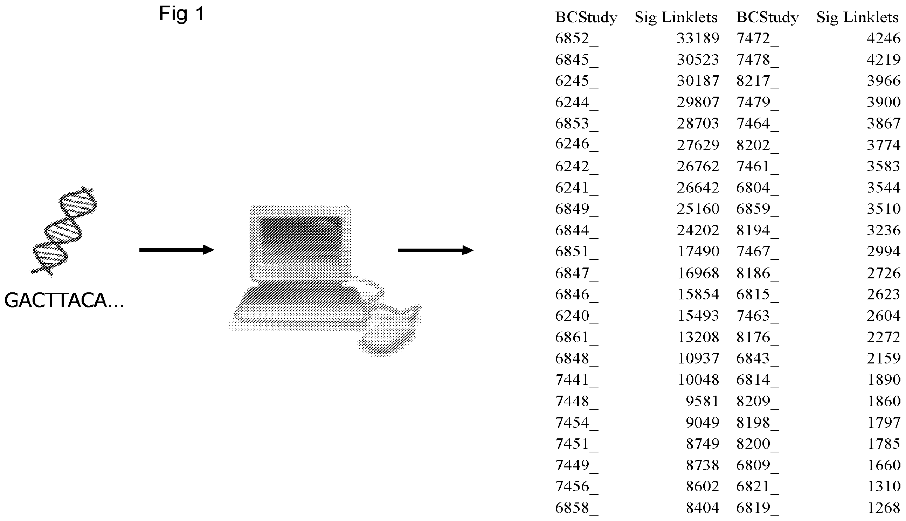

Fig. 1 is a cartoon illustrating the basic concept of the disclosed method, where polynucleotide sequence data from immune system cells is processed using software designed to sort and count the sequences, rank them by frequency numbers, generate p values, and other criteria, to produce a diagnostic signature from the Linklets designated as "Significant Linklets."

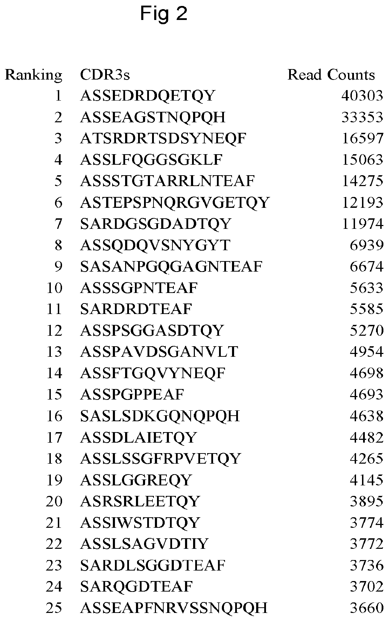

Fig. 2 is a table illustrating the ranking of CDR3s by number of clones present in a sample. Typically, a sequencing result from one sample will generate as many as 400,000 CDR3s. Each CDR3 is associated with a read count; due to the semi-quantitative nature of the amplification method (arm-PCR), the read count also reflects the relative abundance of the clone. Software analysis removes the errors and ranks the CDR3s to produce an output file as shown in the table.

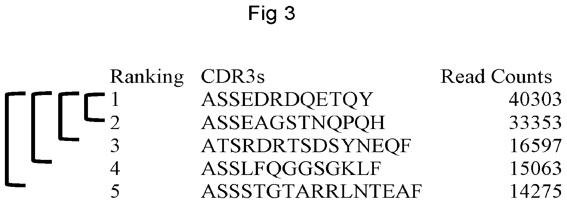

Fig. 3 is a table illustrating how Linklets are detected in the sequences obtained from blood samples. CDR3 sequences were tallied to provide a list of the CDR3 sequences that are present in the highest numbers in a blood sample. Among those CDR3 sequences, Linklets represent pairs of CDR3s that are present within the same sample - at a level that is higher than a designated cutoff level.

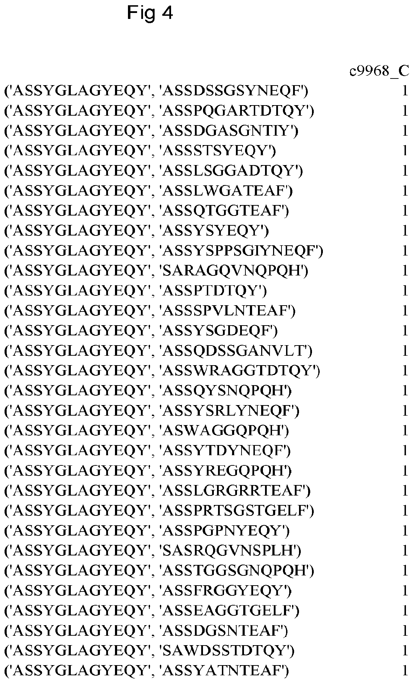

Fig. 4 is a representative list of some of the Linklets identified during a breast cancer study (comparison between Linklets detected in two study groups - a first group of subjects who had been diagnosed with breast cancer and a second group of subjects who were designated as healthy controls).

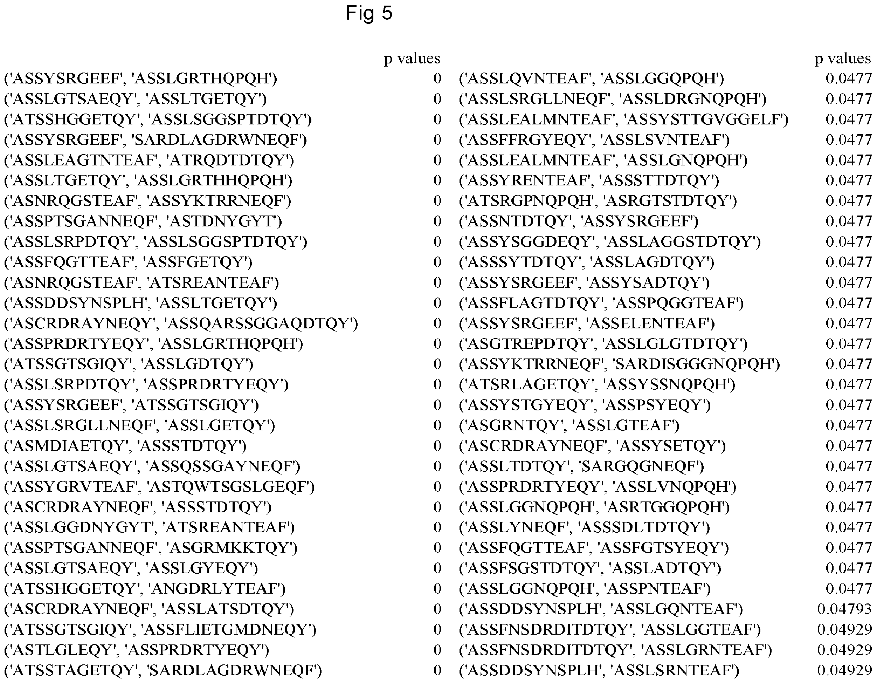

Fig. 5 lists Public Linklets detected in the breast cancer study, based on their p values. The top ranked Linklet, for example, is the CDR3 pair 'ASSYSRGEEF' and 'ASSLGRTHQPQH', and this Linklet was shared in 32 of the 98 breast cancer patient samples, while only 1 of the 106 control samples had it. The p value was 0. Only those Linklets with a p value < 0.05 are included in the final list that represents a breast cancer diagnostic signature. A total of 101,902 Significant Linklets were identified.

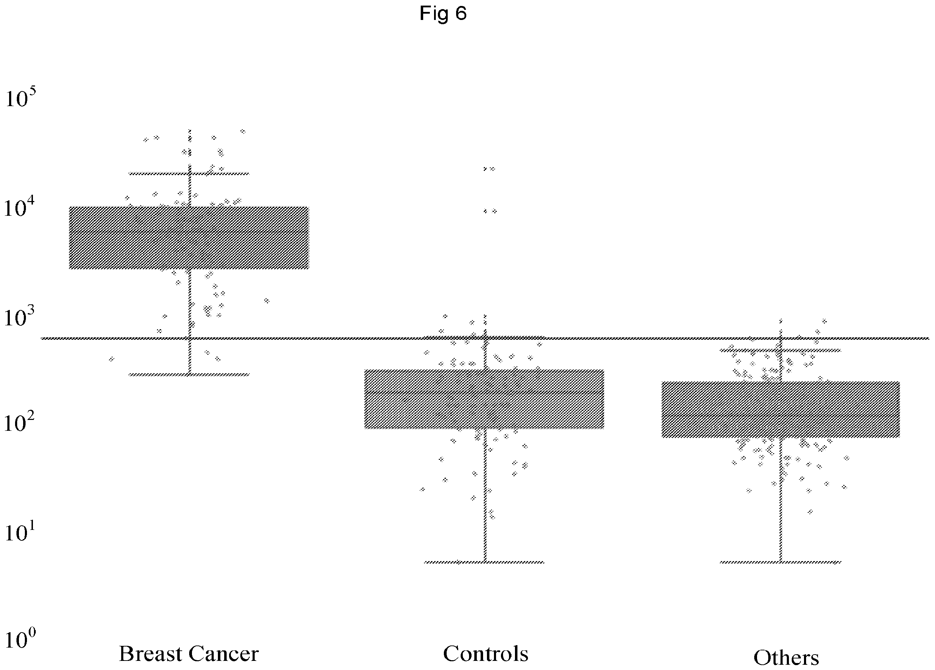

Fig. 6 is a scatter plot illustrating results for subjects in three groups: control, breast cancer, and CMV (cytomegalovirus). A receiver operating characteristic (ROC) curve analysis suggested a cutoff value of 600 DSLs. Of the 103 breast cancer patients, 98 had more than 600 Significant Linklets; of the 110 controls, only 7 had more than 600 Significant Linklets. The diagnostic sensitivity and specificity were therefore 95% and 93%, respectively. When 188 non-breast cancer samples (from patients enrolled in a CMV study) were studied against the breast cancer disease signature, only 3 samples were false positive with more than 600 Significant Linklets, giving a specificity of 98.4%.

### Detailed Description

The present disclosure generally pertains to a method for developing diagnostic tests that are based on the immune response and the resulting immune repertoire. The presently disclosed method increases the signal and reduces the background to allow the identification of shared CDR3s that can be used to produce a disease signature. The presently disclosed method may be used to develop a diagnostic test for different diseases including, but not limited to, cancer, autoimmune disease, inflammatory disease and infectious disease.

As used herein, "disease" includes diagnosed disease and other disruptions, diagnosed and undiagnosed, to the normal health of a subject.

As used herein, "healthy" means not currently exhibiting symptoms of, and not currently diagnosed with, a disease.

As used herein, an "immune repertoire" comprises the functionally diverse T and B cells of a subject.

As used herein, a "Linklet" is a pair of unique CDR3s that are present in the same sample. When two or more people share a particular Linklet, it is a "Public Linklet." If a Linklet is only detected in one subject, it is a "Private Linklet." Public Linklets come from Public CDR3s (i.e., CDR3s that are detected in more than one subject). Generally speaking, each subject's repertoire is largely "private" and only a small percent of that subject's immune repertoire represents shared CDR3s. Public Linklets are therefore present at a much lower level than are Private Linklets, a fact that makes identification of disease signatures more difficult. It is therefore important to utilize an approach that reduces the background to allow identification of the significant CDR3 repertoire that constitutes one or more disease signatures.

As used herein, "sample" comprises blood and tissue. In certain embodiments, blood is peripheral blood collected from a subject. In certain embodiments, tissue is a biopsy obtained from a subject.

As used herein, "subject" means a human or animal.

In certain embodiments, the presently disclosed method reduces the background through the use of "Positive Linklets." When two CDR3 sequences, A and B, are sequenced, quantitative information is also obtained, represented by the read counts. If the immune repertoire amplification method used is semi-quantitative (arm-PCR), and if CDR3-A is expressed in a sample at a higher level than CDR3-B, more sequence read counts will be obtained for A than for B. In such a scenario, the A-B pair is designated as a Positive Linklet, whereas the B-A pair would be designated as a Negative Linklet. In certain embodiments of the presently disclosed method, only the Positive Linklets are used for further analysis. Use of Positive Linklets enriches the diagnostic signal, because it helps to filter out experimental noise.

Biologically, more than one antigen or epitope is generally associated with a particular disease. Therefore, relevant T and B cell receptors would generally appear in patients' samples as clusters. The quantitative information provided by Positive Linklets (and Negative Linklets) may reflect the disease-specific antigen expression profile.

Experimental procedures may introduce additional "noise" in the data generated. For example, it is a common practice to pool many samples from different subjects into one sequencing run to reduce the cost (the immune repertoire being amplified separately using barcoded primers). However, if a CDR3 is very dominant in one sample, it will appear on the sequencing chip multiple times. That dominant clone will have a 1/8,000 chance to be assigned to the wrong barcode and be "shared" by all the other samples in the same run. If CDR3 is used as the basic analytical unit, these "contaminated" sequences would be considered as biologically shared and be used as diagnostic signals. Using Linklets allow these noises to be filtered out, because those incorrectly assigned CDR3s are usually at very low frequency, and the likelihood that they will be part of one or more Positive Linklets is reduced (with a higher likelihood that they will form Negative Linklets). By considering only Positive Linklets, the noise can be filtered out. Also, if only the top ranked CDR3s from a sample are used (such as, for example 5,000, or between 1,000 and 50,000 of the top ranked CDR3s), those incorrectly assigned CDR3s usually will not be considered, due to their low frequencies.

When a group of Public Linklets are found to be associated to a higher degree with a group of subjects who have a particular disease in common, those Public Linklets can be treated as disease-specific Linklets, or "Significant Linklets." A group of Significant Linklets associated with a particular disease can therefore constitute a "disease signature." Therefore, if a subject's sample is found to have statistically significant overlap with the disease signature, a diagnosis of such disease can be made for that subject.

The presently disclosed method comprises the following steps: (1) gathering samples from subjects assigned to a patient group and a control group; (2) amplifying and sequencing an immune repertoire for each sample; (3) identifying the unique CDR3 sequences from each sample's immune repertoire; (4) tallying the number of times an individual (unique) CDR3 sequence is detected in the immune repertoire, thereby identifying those clones that are dominant (determined by ranking them in order of highest frequency of occurrence to lowest frequency of occurrence); (5) comparing the immune repertoires of the subjects to identify CDR3s that are shared between at least two subjects ("Public CDR3s"); (6) ranking the Public CDR3s based on their frequencies of occurrence; (7) generating a list of Positive Linklets from the top-ranked CDR3s; (8) filtering out the Private Linklets and retaining the Public Linklets; and (9) identifying Public Linklets that are associated with patients in the target disease group, but not with the control group.

In certain embodiments, at least about 100 subjects are assigned to each group. In certain embodiments, the immune repertoire is amplified using the arm-PCR method (described in WO2009/124293). In certain embodiments, the top 5,000 clones are identified as dominant. In certain embodiments, the list of Positive Linklets includes the top-ranked 1,000 to 20,000 CDR3s. In certain embodiments, the confidence value for Public Linklets associated with patient in the target disease group, but not with the control group, is p < 0.05.

The list of Public Linklets that are associated with patients (disease associated Linklets, or DSLs), but not with the control group, constitutes a group that is designated as "Signature Linklets." In certain embodiments, a signature may be obtained by analyzing about 100 patients and an equal number of controls. A cutoff Disease Signature Linklet (DSL) value is then determined. Unknown samples may be tested by sequencing followed by counting the DSLs. If the DSL number meets or exceeds the cutoff, a diagnosis is made for a particular disease.

For example, in certain embodiments to obtain the sequence data for analysis, whole blood from a subject (e.g., human or animal, disease group or control (healthy)) is treated with Ficoll® to extract peripheral blood mononuclear cells, or PBMCs, to get the highest concentration of lymphocytes. Each type of lymphocyte has a specific identifier called a CD marker, or cluster of differentiation marker, which is numbered. For example, cytotoxic T-cells have a CD8 marker, and helper T-cells have a CD4 marker. Magnetic beads, which have been labeled with a specific anti-CD marker, can be added to the cell suspension. After applying the column to a magnetic field, the bead bound cells will be trapped, or positively-selected, while allowing the other cell types to flow through. The flow through, or negatively-selected cell suspension, can be used to further isolate other cell populations in downstream applications. In other embodiments, the sample may be tissue, which can be processed, using methods known in the art, to isolate lymphocytes.

Since there are sub-populations within certain populations of T cells (e.g., regulatory T cells are a subpopulation of helper T-cells), if sub-populations need to be separated, release reagents can be added to the CD4-bead-bound cells to release the bead, so that another magnetic bead can bind to the cell. In the case of regulatory T-cells, for example, a CD25+ selection microbead can be added to the cell suspension to extract the regulatory T-cell population from the helper T-cell population.

Polynucleotide isolation (RNA or DNA) can be performed by means known to those of skill in the art (see, e.g., Murray, BMC Res Notes. 2013 Nov 1;6:440). Amplification of sequences may be performed using the method described in WO2009/124293 (arm-PCR), which provides the sensitivity and specificity that is necessary to achieve superior results in the presently disclosed method.

Sequencing may also be performed using methods known in the art. Given the numbers of sequences that must be determined, high-throughput sequencing methods are generally employed, such as, for example, Illumina's Next-Generation Sequencing, using Illumina sequencing primers.

Large amounts of data are generated and must be analyzed and manipulated as a result of the sequencing, tallying the number of times a particular sequence (representing an individual clone) occurs, ranking the clones in order based on frequency of occurrence, and other analyses described herein. This is most conveniently and effectively performed using sequence data analysis programs. One such program is CDR3 Algebra, which does the sorting, ranking, and pairing for the researcher. Statistical analysis, such as calculation of p values, can also be performed using such programs.

The presently disclosed method lends itself to the development of diagnostic tests for a variety of diseases including, but not limited to, cancer, autoimmune disease, bacterial infections, viral infections, and fungal infections, thereby giving researchers and clinicians a valuable tool for the diagnosis and study of a disease of interest.

The presently disclosed method can be further described by means of the following non-limiting examples.

### Examples

### Isolation of Peripheral Blood Mononuclear Cells (PBMCs) from Whole Blood

Whole blood from healthy subjects (control group) and patients previously diagnosed with breast cancer (patient group) was diluted with PBS buffer at 2-4x the original volume. 10 mL of whole blood collected in sodium heparin was transferred to a 50 mL conical tube and diluted with buffer to the 35 mL line. Diluted cell suspension (35 mL) was carefully layered over 15 mL of Ficoll-Paque® in a separate 50 mL conical tube. The tube was centrifuged at 400 x g for 30 minutes at 20 degrees Celsius in a swing bucket rotor with no brake.

The upper layer containing PBS buffer and plasma was carefully aspirated to remove it. The cloudy mononuclear cell layer was carefully transferred to a fresh 50 mL conical tube. The tube was then filled with buffer to the 50 mL mark and centrifuged at 300 x g for 20 minutes at 20 degrees Celsius. The clear supernatant was removed and the cell pellet was re-suspended in 8 mL of buffer.

### Isolation of Monocytes from Isolated PBMCs

Cells were counted using a hemocytometer and the sample centrifuged at 300 x g for 10 minutes at room temperature. The supernatant removed by aspiration. Cells were resuspended in 80 µL of buffer per 10⁷ cells.

Twenty microliters of CD14 Microbeads were added per 1 x 10⁷ cells, and mixing was performed by gently pipetting up and down. The microbead/cell mixture was incubated at 4°C for 15 minutes. Cells were washed by adding 2 mL of buffer per 1 x 10⁷ cells and were then centrifuged at 300 x g for 10 minutes.

The supernatant was aspirated completely and was resuspended in buffer (10⁸ cells in 500 uL of buffer). An LS magnetic column was placed on the magnet and washed with 3 mL of buffer. Flow-through buffer was discarded. Cell suspension was applied to the column and unlabeled cells that pass through were collected in a labeled 15 mL conical tube. The column was washed 3 times with 3 mL of buffer, with new buffer added only when the column reservoir was empty.

A new, clean 15 mL conical tube labeled "Monocyte" was placed under the column and the column was removed from the magnet. Buffer (5 mL) was pipetted into the column and the magnetically labeled cells were immediately flushed out by firmly pushing the plunger into the column.

Both tubes were centrifuged for 10 minutes at 300 x g, and the supernatant completely aspirated. For the tube labeled "Monocyte", the cells were re-suspended in 2 mL of buffer. Twenty microliters were pipetted out to be used for the cell counting protocol, and the tube centrifuged at 300 x g for 10 minutes. Cells were resuspended in 500 µL of RNAprotect® and stored at 4°C for later extraction of RNA. For the tube labeled "CD14-", the cells were re-suspended in 80 µL of buffer per 10⁷ cells.

### RNA Extraction

Cells were centrifuged for 3 minutes at 3,000 rpm at 20°C, supernatant was removed, and the cell pellet was loosened by flicking the tube. BME Buffer (350 µL) was added to the sample, and the cell pellet was dissolved completely by vortexing.

The sample was transferred to a QIAshredder column and homogenized by centrifuging for 2 minutes at 10,000 rpm. The column was discarded, and the flow through was saved. Ethanol (70%, 350 µL) was added to the flow through and the sample was mixed by pipetting. The sample (700 µL) was transferred to an RNeasy® spin column and placed in a 2 ml collection tube. The sample was centrifuged for 15 seconds at 10,000 rpm. Flow through was discarded. In cases where there was more than 700 µL of sample, this step was repeated using the same column.

700 µL of Buffer RW1 was added to the spin column and the sample was centrifuged at 10,000 rpm for 15 seconds, discarding the flow through. 500 µL of Buffer RPE was added to the spin column, and the sample was centrifuged at 10,000 rpm for 15 seconds, discarding the flow through.

500 µL of Buffer RPE was added to the spin column and the sample was centrifuged for 2 minutes at 10,000 rpm. The spin column was placed in a new 2 mL collection tube and was centrifuged for 1 minute at 10,000 rpm to dry the column membrane. The spin column was placed in a new 1.5 mL collection tube. 25 µL of RNase-free water was added to all samples except for samples containing isolated regulatory T cells. To regulatory T cell samples, 20 µL of RNase-free water was added. The sample was allowed to sit at room temperature for 1 minute. The sample was centrifuged for 1 minute at 10,000 rpm and the column was discarded.

### Amplification of CDR3 Sequences Using Polymerase Chain Reaction

PCR amplification of CDR3 sequences was performed using the arm-PCR method disclosed in WO2009/124293 (Han). A minimum of 100 ng of RNA or gDNA (depending on the reagent system selected) with a 260/280 of 1.8 or greater is generally recommended as the starting material to obtain the best diversity of the arm-PCR immune repertoire library. During the first round of PCR, nested gene specific primers targeting each of the V and J (or C) genes were used. The forward primers, Fₒ (forward-out) and Fᵢ (forward-in), targeted the V genes. The reverse primers, Rₒ (reverse-out) and Rᵢ (reverse-in), targeted each of the J or C genes. The Fᵢ and Rᵢ primers also included sequencing adaptors B and A, respectively, for the Illumina® platforms (HiSeq, MiSeq and GAIIx) for paired-end sequencing. The second round of PCR was carried out using communal (common) primers B and A. After gel purification, the resulting product was ready for high throughput sequencing with the Illumina® platforms. The first round of PCR introduced barcodes and sequencing primers into the PCR products.

The exponential phase of the amplification was achieved by the communal primers in the second round of PCR; therefore, the target immune repertoire was amplified evenly and semi-quantitatively, without introducing additional amplification bias.

### Identification of Breast Cancer Signature

A total of 213 samples were collected, including 103 from breast cancer patients and 110 from controls. A total of 14,666,172 CDR3s were identified from the 213 samples, averaging 68,855 CDR3s from each sample. 8,301,648 unique CDR3s were found from the 213 samples. After removing the private CDR3s, a total of 98,076 public (i.e., shared by at least two subjects) and dominant (i.e., ranked within the top 5,000 CDR3s in each sample) CDR3s were identified from the 213 samples, using iRepertoire (Huntsville, Alabama USA) software available through the company website (e.g., CDR3 Algebra). A total of 287,198,206 Positive Linklets were generated from the 213 samples, averaging 1,003,236 Linklets from each sample. After removing Private Linklets, 16,921,605 Linklets remained that were shared with at least one other person. For each shared Linklet, a p value was obtained to identify those preferentially shared among patients. A total of 117,069 Linklets were identified as Significant Linklets with p < 0.05, providing a "signature" for the diseases. A total of 6,171 CDR3s contributed to the 117,069 disease signature Linklets. Using a cutoff value of 600 Significant Linklets, 95% of breast cancer could be diagnosed, with 93.6% of specificity. When 188 non-breast cancer samples were studied, only three samples were false positive (having more than 600 DSLs), giving a specificity of 98.4%.

The presently disclosed method increases the signal and reduces the background to allow the identification of shared CDR3s that can be used to produce a disease signature, which otherwise is not possible using conventional methods. As a result, the presently disclosed method has the benefit of allowing the development of a diagnostic test for different diseases including, but not limited to, cancer, autoimmune disease, inflammatory disease and infectious disease.

The methodologies and the various embodiments thereof described herein are exemplary. Various other embodiments of the methodologies described herein are possible.

## Claims

1. A method for developing a diagnostic test for a particular disease, the method comprising the steps of:
on a collected sample from each of multiple subjects in a patient group and a control group, wherein the patient group comprises subjects who have the same disease and the control group comprises subjects who are healthy;
amplifying and sequencing the immune repertoire of each subject in each of the two groups to identify each unique CDR3 sequence present in the samples and to determine the frequency of occurrence of each unique CDR3 sequence;
identifying CDR3 sequences that are shared between at least two subjects in each of the control group and the patient group;
ranking the CDR3 sequences by order of frequency of occurrence;
identifying the Linklets from each group; and
identifying the Linklets that are associated to a statistically significant degree with the patient group to provide a disease signature.

2. The method of claim 1, wherein the sample is blood.

3. The method of claim 1, wherein the sample is tissue.

4. The method of claim 1, wherein at least about 1,000 CDR3 sequences are identified that are shared between at least two subjects in each of the control group and the patient group.

5. The method of claim 1, wherein at least about 10⁶ Linklets from each group are identified.

6. The method of claim 1, wherein a minimum number of Linklets associated with the disease signature is established as a diagnostic number, so when at least that number of Linklets are identified in a subject's blood sample, that subject is diagnosed as having the disease.

7. The method of claim 6, wherein the diagnostic number is at least about 500.

8. A method for developing a diagnostic test for a particular disease, the method comprising the steps of:
on a collected blood sample from each of multiple subjects in a patient group and a control group, wherein the patient group comprises subjects who have the same disease and the control group comprises subjects who are categorized as healthy;
amplifying and sequencing the immune repertoire of each subject in each of the two groups to identify each unique CDR3 sequence present in the blood samples and to determine the frequency of occurrence of each unique CDR3 sequence;
identifying at least about 1,000 CDR3 sequences that are shared between at least two subjects in each of the control group and the patient group;
ranking the at least about 1,000 CDR3 sequences by order of frequency of occurrence;
identifying at least about 10⁶ Linklets from each group; and
identifying the Linklets that are associated to a statistically significant degree with the patient group to provide a disease signature;
wherein a minimum number of Linklets associated with the disease signature is established as a diagnostic number, so when at least that number of Linklets are identified in a subject's blood sample, that subject is diagnosed as having the disease.

## Patentansprüche

1. Verfahren zum Entwickeln eines Diagnosetests für eine bestimmte Krankheit, wobei das Verfahren die folgenden Schritte umfasst:
auf einer von jedem von mehreren Probanden in einer Patientengruppe und einer Kontrollgruppe genommenen Probe, wobei die Patientengruppe Probanden umfasst, welche dieselbe Krankheit aufweisen, und die Kontrollgruppe Probanden umfasst, welche gesund sind;
Verstärken und Sequenzieren des Immunrepertoires von jedem Probanden in jeder von den zwei Gruppen, um jede in den Proben vorhandene eindeutige CDR3-Sequenz zu identifizieren und die Häufigkeit des Auftretens von jeder eindeutigen CDR3-Sequenz zu bestimmen;
Identifizieren von CDR3-Sequenzen, welche zwischen zumindest zwei Probanden in jeder von der Kontrollgruppe und der Patientengruppe geteilt werden;
Klassifizieren der CDR3-Sequenzen nach Reihenfolge der Häufigkeit des Auftretens;
Identifizieren der Linklets von jeder Gruppe; und
Identifizieren der Linklets, welche zu einem statistisch signifikanten Grad mit der Patientengruppe verknüpft sind, um eine Krankheitssignatur bereitzustellen.

2. Verfahren nach Anspruch 1, wobei die Probe Blut ist.

3. Verfahren nach Anspruch 1, wobei die Probe Gewebe ist.

4. Verfahren nach Anspruch 1, wobei zumindest etwa 1.000 CDR3-Sequenzen identifiziert werden, welche zwischen zumindest zwei Probanden in jeder von der Kontrollgruppe und der Patientengruppe geteilt werden.

5. Verfahren nach Anspruch 1, wobei zumindest etwa 10⁶ Linklets von jeder Gruppe identifiziert werden.

6. Verfahren nach Anspruch 1, wobei eine Mindestanzahl von Linklets, welche mit der Krankheitssignatur verknüpft sind, als eine Diagnoseanzahl eingeführt wird, sodass wenn zumindest diese Anzahl von Linklets in der Blutprobe eines Probanden identifiziert wird, dieser Proband als an dieser Krankheit leidend diagnostiziert wird.

7. Verfahren nach Anspruch 6, wobei die Diagnoseanzahl zumindest etwa 500 ist.

8. Verfahren zum Entwickeln eines Diagnosetests für eine bestimmte Krankheit, wobei das Verfahren die folgenden Schritte umfasst:
auf einer von jedem von mehreren Probanden in einer Patientengruppe und einer Kontrollgruppe genommenen Blutprobe, wobei die Patientengruppe Probanden umfasst, welche dieselbe Krankheit aufweisen, und die Kontrollgruppe Probanden umfasst, welche als gesund kategorisiert sind;
Verstärken und Sequenzieren des Immunrepertoires von jedem Probanden in jeder von den zwei Gruppen, um jede in den Blutproben vorhandene eindeutige CDR3-Sequenz zu identifizieren und die Häufigkeit des Auftretens von jeder eindeutigen CDR3-Sequenz zu bestimmen;
Identifizieren von zumindest etwa 1.000 CDR3-Sequenzen, welche zwischen zumindest zwei Probanden in jeder von der Kontrollgruppe und der Patientengruppe geteilt werden;
Klassifizieren der zumindest etwa 1.000 CDR3-Sequenzen nach Reihenfolge der Häufigkeit des Auftretens;
Identifizieren von zumindest etwa 10⁶ Linklets von jeder Gruppe; und
Identifizieren der Linklets, welche zu einem statistisch signifikanten Grad mit der Patientengruppe verknüpft sind, um eine Krankheitssignatur bereitzustellen;
wobei eine Mindestanzahl von Linklets, welche mit der Krankheitssignatur verknüpft sind, als eine Diagnoseanzahl eingeführt wird, sodass wenn zumindest diese Anzahl von Linklets in der Blutprobe eines Probanden identifiziert wird, dieser Proband als an dieser Krankheit leidend diagnostiziert wird.

## Revendications

1. Procédé de mise au point d'un test diagnostique pour une maladie particulière, le procédé comprenant les étapes suivantes :
sur un échantillon collecté auprès de chacun de multiples sujets dans un groupe de patients et un groupe témoin, dans lequel le groupe de patients comprend des sujets qui ont la même maladie et le groupe témoin comprend des sujets qui sont sains ;
amplification et séquençage du répertoire immunitaire de chaque sujet dans chacun des deux groupes pour identifier chaque séquence CDR3 unique présente dans les échantillons et pour déterminer la fréquence de survenue de chaque séquence CDR3 unique ;
identification des séquences CDR3 qui sont partagées entre au moins deux sujets dans chacun du groupe témoin et du groupe de patients ;
classement des séquences CDR3 par ordre de fréquence de survenue ;
identification des linklets dans chaque groupe ; et
identification des linklets qui sont associés à un degré statistiquement significatif avec le groupe de patients pour fournir une signature de maladie.

2. Procédé selon la revendication 1, dans lequel l'échantillon est du sang.

3. Procédé selon la revendication 1, dans lequel l'échantillon est un tissu.

4. Procédé selon la revendication 1, dans lequel au moins environ 1 000 séquences CDR3 sont identifiées qui sont partagées entre au moins deux sujets dans chacun du groupe témoin et du groupe de patients.

5. Procédé selon la revendication 1, dans lequel au moins environ 10⁶ linklets dans chaque groupe sont identifiés.

6. Procédé selon la revendication 1, dans lequel un nombre minimal de linklets associés à la signature de maladie est défini en tant que nombre de diagnostic, de telle sorte que lorsqu'au moins ce nombre de linklets sont identifiés dans un échantillon de sang d'un sujet, ce sujet est diagnostiqué comme ayant la maladie.

7. Procédé selon la revendication 6, dans lequel le nombre de diagnostic est au moins environ 500.

8. Procédé de mise au moins d'un test diagnostique pour une maladie particulière, le procédé comprenant les étapes suivantes :
sur un échantillon de sang collecté auprès de chacun de multiples sujets dans un groupe de patients et un groupe témoin, dans lequel le groupe de patients comprend des sujets qui ont la même maladie et le groupe témoin comprend des sujets qui sont catégorisés sains ;
amplification et séquençage du répertoire immunitaire de chaque sujet dans chacun des deux groupes pour identifier chaque séquence CDR3 unique présente dans les échantillons de sang et pour déterminer la fréquence de survenue de chaque séquence CDR3 unique ;
identification d'au moins environ 1000 séquences CDR3 qui sont partagées entre au moins deux sujets dans chacun du groupe témoin et du groupe de patients ;
classement des au moins environ 1000 séquences CDR3 par ordre de fréquence de survenue ;
identification d'au moins environ 10⁶ linklets dans chaque groupe ; et
identification des linklets qui sont associés à un degré statistiquement significatif avec le groupe de patients pour fournir une signature de maladie ;
dans lequel un nombre minimal de linklets associés à la signature de maladie est défini en tant que nombre de diagnostic, de telle sorte que lorsqu'au moins ce nombre de linklets sont identifiés dans un échantillon de sang d'un sujet, ce sujet est diagnostiqué comme ayant la maladie.
